# EUROPEAN PATENT SPECIFICATION

(11) **EP 2 364 218 B1**
(45) Date of publication and mention of the grant of the patent: **15.04.2020**
(21) Application number: 09760034.0
(22) Date of filing: 30.10.2009
(51) Int. Cl.: G01N 21/03, G01N 21/35, G01N 21/3581, G01N 21/359, G01N 21/47, G01N 21/64, G01N 21/65, C12Q 1/04, G01N 33/68

(54) **CONTAINER FOR THE ISOLATION AND IDENTIFICATION OF MICROORGANISMS**
BEHÄLTER ZUR ISOLIERUNG UND IDENTIFIKATION VON MIKROORGANISMEN
RECIPIENT POUR L'ISOLATION ET L'IDENTIFICATION DE MICRO-ORGANISMES

(30) Priority: 31.10.2008 US 110187 P
(43) Date of publication of application: 14.09.2011
(73) Proprietor: Biomerieux, Inc, Durham, NC 27712 (US)
(72) Inventor: WALSH, John, Durham NC 27713 (US); HYMAN, Jones, Wake Forest NC 27587 (US); RONSICK, Christopher, Durham NC 27712 (US); LINK, John, Durham NC 27712 (US); ROBINSON, Ron, Durham NC 27705 (US); WILSON, Mark, Hillsborough NC 27278 (US)
(74) Representative: Murgitroyd & Company
(86) International application number: PCT/US2009/005888
(87) International publication number: WO 2010/062353

(56) References cited:
- EP-A2- 0 901 821
- EP-A2- 1 346 773
- WO-A1-2004/043601
- WO-A2-02/21108
- WO-A2-02/068580
- US-A- 4 007 639
- US-A- 4 021 352
- US-A- 4 212 948
- US-A- 4 687 479
- US-A- 5 242 660
- US-A- 5 257 984
- US-A- 5 456 885
- US-A- 5 736 398

## Description

### FIELD OF THE INVENTION

The present invention is directed to a separation device for the separation of microorganisms. In particular, the device of the present invention can be used to separate microorganisms for characterization and/or identification.

### BACKGROUND OF THE INVENTION

The detection of pathogenic microorganisms in biological fluids should be performed in the shortest possible time, in particular in the case of septicemia for which the mortality remains high in spite of the broad range of antibiotics which are available to doctors. The presence of biologically active agents such as a microorganism in a patient's body fluid, especially blood, is generally determined using blood culture bottles. Bloodstream infections are associated with high morbidity and mortality, yet current diagnostic methods, of culture followed by biochemical identification and antibiotic susceptibility testing, can take several days to perform. Typically, empiric therapy is initiated based on clinical symptoms, and test results only impact clinical decisions when the initial therapy fails. The ability to characterize bloodstream infections within the first few hours, preferably within an hour, after a positive blood culture result would significantly boost the clinical relevance of the diagnostic information provided. Molecular amplification methods have been proposed to fill this need, but serious challenges to this approach remain. The positive blood culture broth itself represents a naturally amplified population of microorganisms with potential for use in a variety of rapid, identification (ID) tests.

Traditional automated phenotypic ID tests, such as the Vitek®, Phoenix™ and Microscan® systems, or manual phenotypic tests such as API require that microorganisms be in an appropriate growth phase and free of interfering media and blood products in order to provide robust results. These systems use colonies grown from the positive broth for 18-24 hours on plated media. However, in an effort to obtain faster results, some laboratories have reported using these systems with microorganisms isolated from positive blood culture bottles. These direct-from-the-bottle tests are not appropriate for all microorganisms (e.g., Gram-positive cocci), are not validated by the test manufacturers, and generally take 3-8 hours to provide results. Faster and more broadly specific tests are urgently needed in order to provide the physician with clinically relevant results within the first few hours, preferably within an hour, after a positive culture result.

Optical spectroscopy methods, such as intrinsic fluorescence (IF), infrared spectroscopy (FTIR), or Raman spectroscopy, and mass spectrometry methods such as MALDI-TOF, have the potential to allow for identification of microorganisms very quickly, but may encounter interference from the many highly fluorescent and absorptive compounds present in liquid microbiological culture media and in clinical samples such as blood or combinations thereof. The most commonly employed methods for recovering microorganisms directly from positive blood culture broth are two-step differential centrifugation and centrifugation in a serum separator tube. However, these methods have several drawbacks. The resultant microbial preparation often contains contaminating red blood cells, platelets, lipid particles, plasma enzymes and cellular debris, which can cause poor results in traditional phenotypic ID tests. These methods are also very labor-intensive and unsafe due to steps which can result in aerosol exposure of potentially dangerous pathogens to the user. Simple, safe and reliable methods are needed to isolate microorganisms from blood culture broth and other complex samples that are free of these interfering materials and compatible with rapid identification technologies.
WO 02/068580 discloses an integrated filtration and detection device for collecting and detecting the growth of microorganisms in a specimen, including a container defining a chamber therein. The container has an inlet and an outlet in fluid communication with the chamber. A filter is mounted in the chamber between the inlet and the outlet. A sensor is mounted in the chamber. The sensor is operative to exhibit a change in a measurable property thereof upon exposure to changes in the chamber due to microbial growth.
US 4,687,479 discloses a blood storage device consisting of a blood extraction tubule and a removable cover which closes its upper opening.
WO02/21108A2 discloses a method and system for separating microorganisms, especially infectious agents, from a mixture on the basis if isopycnic banding density.
US 4, 212, 948 discloses an apparatus for detecting microbial pathogens employing a cushioning agent.

### SUMMARY OF THE INVENTION

The present invention is directed to a separation device or container that can be used for the separation of microorganisms from a sample that contains or is suspected of containing microorganisms. In accordance with the present invention, the separation device can be used for the separation or pelleting of an unknown microorganism and subsequent interrogation of the separated sample or pellet for characterization and/or identification of the unknown microorganism.

The present invention is directed to a container for isolating and identifying a microorganism in accordance with claim 1, said container comprising:
(a) a lower portion comprising a capillary tube having an internal diameter from about 0.001 to about 0.04 inches (about 0.0254 mm to about 1.016 mm);
(b) an upper internal chamber or reservoir having an internal diameter wider than the internal diameter of said capillary tube;
(c) a middle tapered portion connecting said upper portion to said lower portion;
(d) a density cushion in the container, wherein the density cushion has a homogeneous density in the range of 1.025 to 1.120 g/ml, wherein an intermediate layer of polyethylene beads is placed on top of the density cushion, and wherein the container further contains a selective lysis solution;
(e) an optical window on the bottom or one or more sides of the container and having a thickness of less than 0.10 inches (2.54 mm), said optical window being transparent to at least a portion of the near infrared, visible, and/or ultraviolet light spectrum and wherein said optical window comprises quartz, fused silica, sapphire, acrylic, methacrylate, a cyclic olefin copolymer, a cyclo olefin polymer or any combination thereof; and
(f) a closure or cap.

Using the container of the present invention, the microbial agent can be separated or pelleted at the bottom of the capillary tube located in the separation device or container in the manner described herein. The separated or pelleted microbial agent can be interrogated for characterization and/or identification of the microbial agent.

The separation device can be sealed, for example, the device can be hermetically sealed. Such devices can provide safety advantages when handling potentially infectious agents. The separation device can provide a means to access the separated, isolated, or pelleted microorganism sample, thereby allowing the sample to be removed from the separation device prior to interrogation, or for additional testing.

### BRIEF DESCRIPTION OF THE FIGURES

Figure 1 is a perspective view of a separation device.
Figure 2 is a cross-sectionai view of the separation device of Figure 1.
Figure 3 is a perspective view of a separation device.
Figure 4 is a cross-sectional view of the top portion of the separation device shown in Figure 3.
Figure 5 is a cross-sectional view the bottom portion of the separation device shown in Figure 3. The bottom portion of the separation device is fitted to the lower end of top portion of the separation device of Figure 4.
Figure 6 is a cross-sectional view of the separation device of Figure 3, showing a separated microbial agent in the capillary tube section of the separation device (e.g., the pellet after centrifugation).
Figure 7 is a cross-sectional view of one example of the bottom portion of the separation device of Figure 3. As shown, this example has two indented opposing sides leading to adjacent narrow side walls, thus allowing the separated microbial agent in the capillary tube section to be interrogated from the side of the separation device.
Figure 8 is a schematic illustration of the concentrated microbial agent in the separation device of Figure 6 being interrogated through the bottom of the tube by an interrogation module.
Figure 9 is a perspective view of yet alternative example of the separation device.
Figure 10 is a cross-sectional view of the separation device of Figure 9.
Figure 11 is a perspective view of an alternative cap for the separation device .
Figure 12 shows a photograph of a separation device in accordance with one example. Clearly visible in the photograph are the lysed sample, density cushion and a microorganism pellet.

### DETAILED DESCRIPTION OF THE INVENTION

Methods for the separation, characterization and/or identification of microorganisms have been disclosed in the following commonly assigned U.S. patent applications: (1) serial nos. 12/589,929, entitled "Method for the Isolation and Identification of Microorganisms", filed October 30, 2009; (2) serial nos. 12/589,952, entitled "Method for Separation, Characterization and/or Identification of Microorganisms using Spectroscopy", filed October 30, 2009; (3) serial nos. 12/589,936, entitled "Method for Separation, Characterization and/or Identification of Microorganisms using Mass Spectrometry", filed October 30, 2009; and (4) serial nos. 12/589,976, entitled "Method for Separation, Characterization and/or Identification of Microorganisms using Raman Spectroscopy", filed October 30, 2009. Briefly, these patents disclosed methods for isolating, characterizing and/or identifying microorganisms in a sample. The methods allow for the separation, characterization and/or identification of microorganisms more quickly than prior techniques, resulting in faster diagnoses (*e.g.*, in a subject having or suspected of having septicemia) and identification of contaminated materials (*e.g*., foodstuffs and pharmaceuticals). In these, and other methods of characterizing and/or identifying microorganisms, it is often necessary to provide a separated, isolated, or pelleted microorganism sample for subsequent characterization and/or identification procedures. The present invention discloses a separation device that can be used for the separation, isolation and/or pelleting of microorganisms from a sample. For example, the separation device of the present invention can be used to pellet microorganisms (e.g., by centrifugation) from a liquid culture (e.g., a blood culture). The microorganism pellet can then undergo one or more interrogation steps to provide measurements useful for characterization and/or identification of the microorganism.

The interrogation step can be carried out while the separated, isolated or pelleted microorganism sample remains in the separation device. For example, a sealed separation device (e.g., hermetically sealed device) can be used for the preparation of a separated, isolated or pelleted microorganism sample, and subsequently, the separated, isolated or pelleted microorganism sample can be subjected to a non-invasive interrogation technique to provide data or measurements capable of characterization and/or identification of the microorganism. The separated, isolated, or pelleted microorganism sample can be removed from the separation device prior to interrogation. For example, the separated, isolated or pelleted microorganism can be resuspended in an appropriate buffer and removed (e.g., by pipette) from the device or container. As disclosed herein, the separation device or container may include a lower portion that is capable of being removed, or snapped apart, from the separation device or container (i.e., a removable lower portion). In operation, this lower portion can be snapped off from the separation device or container to provide access to the separated or isolated microorganisms therein.

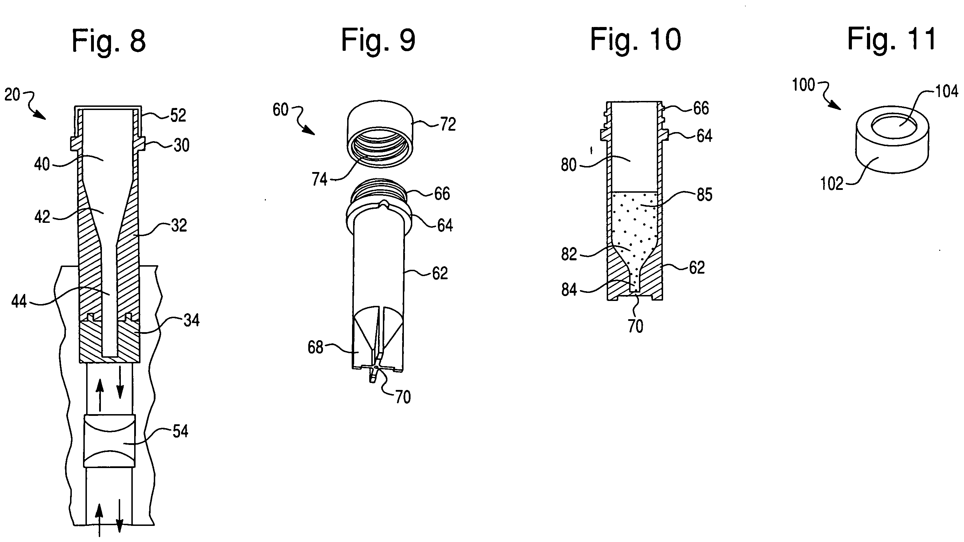

In general, the separation device or container of the present invention may be any device or container useful for the separation, isolation or pelleting of a microorganism from a test sample containing or suspected of containing microorganisms. For example, the separation device or container may comprise a single-, or multi-piece body and a closure or cap. The body of the device can be molded, blow-molded, or formed using other well known techniques in the art. In general, any known plastic, glass, or transparent material, or the like, can be used for the separation device. The separation device will be formed have an opening at one end providing access to the interior of the device or container for loading and/or unloading test samples. The separation device or container can comprise a cylinder shaped body, which is closed at one and open at an opposite one end. Typically, the closure or cap can employ any known mechanism to close or otherwise seal off the interior of the device or container from the outside environment. For example, the closure or cap may be a snap-type lid that is attached to the body of the device or container and that can be snapped over the opening of the device or container to close or seal the interior of the device from the outside environment. Alternatively, the closure can be a threaded cap that can be screwed onto the device or container to close the device/container. As is well known in the art, the cap can have threads on the inner sidewall of the cap that thread or screw onto threads located on an exterior wall of the device or container. The cap can contain one or more rubber O-ring on the inside surface thereof, as is well known in the art. The use of one or more O-rings provides a seal (e.g., a hermetic seal). In another possible example, as shown in Figure 11, the closure or cap 100 may have a pierceable septum 104 capable of being pierced, e.g., by a needle or the like, thereby allowing for the deliver of a test sample into the sealed device or container. The use of a pierceable septum 104 can provide a safety advantage for the user or technician when handling potentially infectious agents and enables automation of the identification method. The pierceable septum 104 also ensures the device or container remains sealed (e.g., hermetically sealed), and thus, provides protection from possible contamination for the separation device.

Test samples that may be subjected to separation, isolation, or pelleting in the separation device or container of the present invention include both clinical and non-clinical samples in which microorganism presence and/or growth is, or may be suspected, as well as samples of materials that are routinely or occasionally tested for the presence of microorganisms. For example, the test sample can be the culture broth from a culture of a clinical or non-clinical specimen sample. Typical specimen samples that may be cultured and subsequently subjected to a separation technique for separation, isolation, or pelleting of microorganisms contained therein, may include, blood, serum, plasma, blood fractions, joint fluid, urine, semen, saliva, feces, cerebrospinal fluid, gastric contents, vaginal secretions, tissue homogenates, bone marrow aspirates, bone homogenates, sputum, aspirates, swabs and swab rinsates, other body fluids, and the like.

In the invention, as described further herein, the separation device or container employs the use of a density cushion for the separation, isolation or pelleting of microorganisms from a test sample. As used herein, the term "density cushion" refers to a solution having a homogenous density throughout. Useful density cushions are further described herein. For example, a test sample known to contain, or that may contain microorganisms can be loaded over a density cushion contained within the device or container, and the container of device centrifuged to isolate or pellet the microorganisms. In accordance with this embodiment, the separation device or container will have sufficient volume to hold a density cushion and a sample. The container may be fitted or can be fitted into a centrifuge rotor. The volume of the container can be from about 0.1 ml to about 25 ml, *e.g.,* from about 1 ml to about 15 ml, *e.g.,* from about 1.5 ml to about 8 ml. If the separation is done on a microscale, the volume of the container can be from about 2 µl to about 100 µl, *e.g.,* from about 5 µl to about 50 µl. According to the invention, as discussed in more detail herein, the separation device or container is preloaded with the density cushion. According to the invention, an intermediate layer (liquid or solid) is placed on top of the density cushion before the sample is laid or layered on top in order to prevent any mixing of the density cushion and the sample. The separation device or container is preloaded with a density cushion and subsequently preloaded with a lysis solution. Useful lysis solutions are disclosed in the commonly assigned U.S. patent applications discussed herein.

In the invention, the device or container has an upper internal chamber or reservoir having a wide diameter to hold the test sample and the majority of the density cushion, and a lower internal chamber or capillary tube having a narrow diameter for collecting the separated, isolated or pelleted microorganisms. The upper internal chamber or reservoir can have an internal diameter of about 0.32 to about 0.40 inches (8.128 to about 10.16 mm), *e.g.,* about 0.34 to about 0.38 inches (8.636 to about 9.652 mm), *e.g.,* about 0.36 inches (9.144 mm). For microscale separations, the internal diameters can be even smaller. For example, the internal diameter of the narrow portion is 0.001 to 0.04 inches (0.0254 to about 1.016 mm), *e.g.,* about 0.002 to about 0.01 inches (0.0508 to about 0.254 mm). The lower internal chamber or capillary tube can have an internal diameter of about 0.04 to about 0.12 inches (1.016 to about 3.048 mm), *e.g.,* about 0.06 to about 0.10 inches (1.524 to about 2.54 mm), *e.g.,* about 0.08 inches (2.032 mm).

The separation device or container has a middle tapered portion or chamber connecting the upper internal chamber or reservoir with the lower internal chamber or capillary tube. The inner sidewalls of the middle tapered portion is tapered, and decreases in diameter, between the upper internal chamber or reservoir with the lower internal chamber or capillary tube. The inner sidewalls of the tapered portion can have an angle of about 20 to about 70 degrees, *e.g.,* about 30 to about 60 degrees. In one embodiment, the lower narrow portion is less than half of the total height of the container, *e.g.,* less than about 40%, 30%, 20%, or 10% of the total height of the container.

The container can be designed such that the separated, isolated, or pelleted microorganisms can be readily recovered from the container after separation, either manually or in an automated manner (so that technicians are not exposed to the container contents). For example, the container can comprise a removable portion or a break-away portion which contains the pellet and which can be separated from the rest of the container. The container can comprise means for access to the pellet after separation, such as one or more ports or permeable surfaces for insertion of a syringe or other sampling device or for drawing off the pellet. In one example, the container can be a tube, *e.g.,* a centrifuge tube. In one example, the container is a stand alone container, *i.e.,* a device for separating a single sample.

The container comprises an optical window through which the interrogation can occur. The optical window may be on the bottom or sides of the container. The window is composed of a material that is transparent to light (*e.g.,* at least a portion of the near infrared (NIR; 700 nm-1400 nm), ultraviolet (UV; 190 nm-400 nm) and/or visible (VIS; 400 nm-700 nm) light spectrum), selected from acrylic, methacrylate, quartz, fused silica, sapphire, a cyclic olefin copolymer (COC) and/or a cyclo olefin polymer (COP) (e.g., Zeonex® (Zeonex®, San Diego, CA)). The entire container can be made of optical window material. In another example, the container may be prepared (*e.g*., molded) from two or more separate parts, such as an optical UV-VIS-NIR transparent component for the optical window and another material (*e.g*., a lower-cost standard molding plastic) to make up the rest of the container. In the invention, the optical window is thin enough to permit spectroscopic interrogation, which will depend on the material of the window. Furthermore, the optical window is as thin as possible to reduce interference with spectroscopic interrogation. For example, the window can have a thickness of less than 0.10 inches (2.54 mm)or 0.05 inches (1.27 mm).

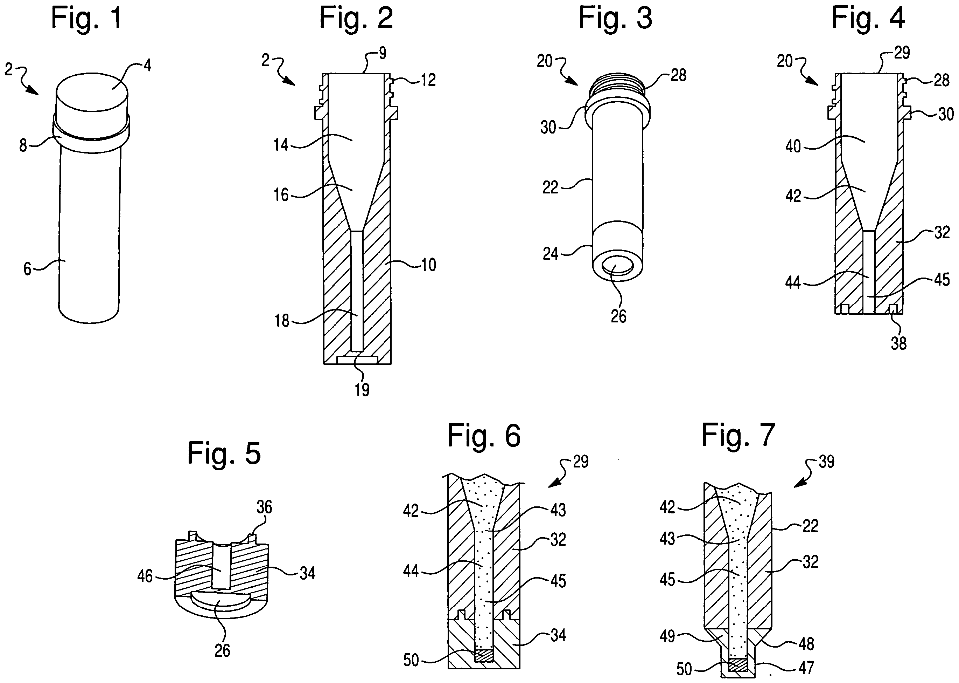

Referring now to the Figures, several possible configurations for the separation device or container will be further illustrated. One possible embodiment of the separation device is shown in Figures 1-2. As shown in Figures 1 and 2, the separation device 2 comprises a lower portion 6, generally having a cylinder shape, and an upper portion defined by an externally projecting ridge structure or ledge 8, an opening 9, and a closure cap 4. The lower portion 6 comprises a container body 10 that encloses an internal chamber comprising an upper reservoir 14, a middle tapered section 16 and a lower capillary tube 18, all arranged around the longitudinal axis of the container. As shown, the middle tapered section 16 connects the wider diameter upper reservoir 14 and the smaller diameter capillary tube 18. In general, the container body 10 can be molded or otherwise formed from any known plastic material known in the art. The externally projecting ridge structure or ledge 8 can function as a stop for the closure cap 4 and/or can provide a feature allowing for improved gripping of the device by a user. The upper portion of the device may also provide threads 12 on the external wall of the device 2 for threading or screwing the closure cap 4 onto the device 2, thereby closing or sealing the internal chamber. The device may further contain a thin optical window 19 through which the interrogation can occur. In one example, the diameter of the optical window 19 can be designed to match a fiber optic cable and facilitate precise coupling of the device to a spectrometer. As previously described, the optical window 19 comprises a section of the device that is composed of a material that is transparent to light, and through which interrogation can occur.

According to the invention, the separation device 2 ispre-loaded with a density cushion 43 (shown e.g., in Figures 6-7) to facilitate the separation, isolation or pelleting of microorganisms or it can be added to the separation device 2 just prior to the loading of the sample to be subjected to the separation step described herein. In the invention, the separation device 2 is preloaded with a density cushion 43 and a lysis solution (not shown) to facilitate sample lysis and separation, isolation or pelleting of microorganisms, as described in the commonly assigned U.S. patent applications referenced herein.

As shown in Figures 3-6 and 8, the separation device 20 can be made of two separate sections, an upper section 22 and a lower section 24, that can be snapped together, or otherwise attached, to form a single separation device 20. The lower section 24 can be removably attached, or permanently attached, to the upper section 22, in general, by any known means in the art. The upper section 22 comprises an upper body 32, generally comprising a cylinder shape, an externally projecting ridge structure or ledge 30, and opening 29. The opening can be closed or sealed using a closure or cap 52 (see Figure 8). The upper body 32 further defines the upper portions of an internal chamber. The internal chamber comprises an upper reservoir 40, a middle tapered section 42 and the upper part 44 of a capillary tube 45, all arranged around the longitudinal axis of the container. The lower body 34 comprises the lower part 46 of the capillary tube 45. When the upper body 32 and lower body 34 are snapped together, or otherwise affixed, they enclose the upper chamber, again comprising an upper reservoir 40, a middle tapered section 42 and a capillary tube 45. As shown, the middle tapered section 42 connects the wider diameter upper reservoir 40 and the smaller diameter capillary tube 45. The lower body 34 further comprises a structure, for example, a protruding ridge 36 formed in on the top of lower body 34, that can be fitted (e.g., snapped or attached) into a corresponding recess 38 in the bottom of the upper body 32. The lower body 34 of the device 20 contains a thin optical window 26 through which the interrogation can occur. As previously described, the optical window 26 comprises a section of the device that is composed of a material that is transparent to light, and through which interrogation can occur. The entire device may be made from a material that is transparent to light, thereby allowing interrogation therethrough. As previously described, the container is designed such that the separated, isolated, or pelleted microorganisms can be readily recovered from the container after separation, either manually or in an automated manner (so that technicians are not exposed to the container contents). For example, the lower section 24 may be removable after a separation step, thereby allowing a user to access the separated or pelleted microorganisms, which will be contained in the lower part 46 of the capillary tube 45, of the lower section 24.

The separation device 20 is pre-loaded with a density cushion 43 (shown e.g., in Figures 6-7) to facilitate the separation, isolation or pelleting of microorganisms. The density cushion can be added to the separation device 20 just prior to the loading of the sample to be subjected to the separation step described herein. In the invention the separation device 20 is preloaded with a density cushion 43 and a lysis solution (not shown) to facilitate sample lysis and separation, isolation or pelleting of microorganisms.

Another example of the lower section of the separation device is shown in Figure 7. The lower section 48 can be removably attached, or permanently attached, to the upper section 22 to form a separation device 39. The upper section 22 and lower section 47 comprises an upper body 32 and a lower body 49, respectively, that define an internal chamber. The internal chamber comprises an upper reservoir (not shown), a middle tapered section 42 and a capillary tube 45. As shown, the lower body 34 further comprises exterior walls that slope inward 48, that result in thin sidewalls on opposite sides of the bottom of the internal capillary tube 45. These thin sidewalls, allow for interrogation of a separated, isolated or pellet microorganism 50 through the side of the separation device.

In yet another example a separation device 60 is shown in Figures 9-10. Referring to these Figures, the separation device 60 consists of a body 62 that defines an upper reservoir 80, a middle tapered section 82 and a lower capillary tube 84. The middle tapered section 82 connects the larger diameter upper reservoir 80 with the lower capillary tube 84. The upper reservoir 80 is accessed via a removable closure or cap 72 that can be threaded or screwed onto threads 66 formed at the top exterior wall of the body 62. In accordance with this embodiment, the lower portion of the body 62 comprises four stabilizing wings 68 used to provide stability to the separation device 60 when standing upright, e.g., on a table. In another example, the four indents on the bottom of the wings 68 create a recessed area for the precise coupling of a fiber optic probe. Centering of the excitation beam in such a way resulted in improved fluorescence reproducibility and reduced contamination of the emission signal by stray scattered light. The separation device 60 further comprises an optical window 70 formed in the body 62 at the bottom of the capillary tube 84. The optical window 70 comprises a small section of reduced thickness on the body 62, through which the separated, isolated, or pelleted microorganism can be interrogated. As described hererin, the optical window 70 can be made from an optically transparent material.

The separation device 60 is pre-loaded with a density cushion 85 (as shown e.g., in Figure 10) to facilitate the separation, isolation or pelleting of microorganisms. The density cushion can be added to the separation device 60 just prior to the loading of the sample to be subjected to the separation step described herein. In the invention, the separation device 60 is preloaded with a density cushion and a lysis solution to facilitate sample lysis and separation, isolation or pelleting of microorganisms.

Figure 8 shows the operation of interrogation of concentrated microbial agent 50 within the separation device 60. The separated, isolated or pelleted microorganisms can be interrogated using any known means in the art (represented here as an interrogation means 54). For examples, as disclosed in co-pending U.S. patent application, serial no. 12/589,929, titled "Method for the Isolation and Identification of Microorganisms", filed October 30, 2009, the interrogation step can be carried out using intrinsic fluorescence spectroscopy, Raman spectroscopy or other optical technique.

The concentrated microbial agent can be interrogated while it is still located within the separation device, or the separated, isolated or pelleted microorganism sample can be removed from the separation device and interrogated, for example, using Mass Spectrometry, as disclosed in co-pending U.S. patent application, serial no. 12/589,952, titled "Method for Separation, Characterization and/or Identification of Microorganisms using Mass Spectrometry", filed October 30, 2009.

As noted hereinabove, the separation, isolation or pelleting step can be carried out to separate the microorganisms from other components of the sample (e.g., non-microorganisms or components thereof) and to concentrate the microorganisms into a separated, isolated or pellet sample that can be interrogated for identification and characterization purposes. The separation or pelleting step does not have to be complete, *i.e.,* it is not required that 100% separation occur. All that is required is that the separation of the microorganisms from other components of the sample be sufficient to permit interrogation of the microorganisms without substantial interference from the other components. For example, the separation can result in a microorganism pellet that is at least about 10, 20, 30, 40, 50, 60, 70, 80, 90, 95, 96, 97, 98, or 99% pure or higher.

As described more fully in the commonly assigned U.S. patent applications discussed herein, the separation can be carried out by a centrifugation step in which a test sample (*e.g.,* a lysed sample) is placed on top of a density cushion in a separation container and the container centrifuged under conditions which allow the microorganisms to be isolated (e.g., the microorganisms can form a pellet at the bottom and/or sides of the container). In accordance with the example, other components of the sample (e.g., non-microorganisms or components thereof that may be present in the sample medium) stay on top of the density cushion or within the top portion of the density cushion. This separation step isolates the microorganisms away from materials in the sample, such as medium, cell debris, and/or other components that might interfere with interrogation of the microorganisms (*e.g.,* by intrinsic fluorescence). In one example, the density cushion also serves to separate live microorganisms from dead microorganisms (which do not pass through the density cushion). The density cushion does not comprise a density gradient, either before or after the centrifugation. In other words, the separation container is not centrifuged for a sufficient amount of time and/or acceleration for the material making up the density cushion to form a density gradient.

The density of the cushion is selected such that the microorganisms in the sample pass through the cushion while other components of the sample (*e.g.,* blood culture broth, cell debris) remain on top of the cushion or do not pass all of the way through the density cushion. The density cushion may also be selected to separate live microorganisms (which pass through the cushion) from dead microorganisms (which do not pass through the cushion). Suitable densities will depend on the material used in the density cushion and on the sample to be separated. The density of the cushion is in the range of 1.025 to 1.120 g/ml, *e.g.,* about 1.030 to about 1.070 g/ml, about 1.040 to about 1.060 g/ml or any range between about 1.025 to about 1.120 g/ml. In another embodiment, the density of the cushion is about 1.025, 1.030, 1.035, 1.040, 1.045, 1.050, 1.055, 1.060, 1.065, 1.070, 1.075, 1.080, 1.085, 1.090, 1.095, 1.100, 1.105, 1.110, 1.115, or 1.120 g/ml.

The material for the density cushion can be any material that has the appropriate density range for the methods of this invention. In one embodiment, the material is colloidal silica. The colloidal silica may be uncoated (*e.g*., Ludox® (W.R. Grace, CT)) or coated, *e.g.,* with silane (*e.g.,* PureSperm® (Nidacon Int'l, Sweden) or Isolate® (Irvine Scientific, Santa Ana, CA)) or polyvinylpyrrolidone (*e.g.,* Percoll™, Percoll™ Plus (Sigma-Aldrich, St. Louis, MO)). In one example, the colloidal silica exhibiting the least interference with spectroscopic interrogation is selected, *e.g.,* the material with the lowest intrinsic fluorescence. The colloidal silica may be diluted in any suitable medium to form the proper density, *e.g.,* balanced salt solutions, physiological saline, and/or 0.25 M sucrose. Suitable densities can be obtained with colloidal silica at a concentration of about 15% to about 80% v/v, *e.g.,* about 20% to about 65% v/v. Another suitable material for density cushions is an iodinated contrast agent, *e.g.,* iohexol (Omnipaque™ NycoPrep™, or Nycodenz®) and iodixanol (Visipaque™ or OptiPrep™). Suitable densities can be obtained with iohexol or iodixanol at a concentration of about 10% to about 25% w/v, *e.g.,* about 14% to about 18% w/v, for blood culture samples. Sucrose can be used as a density cushion at a concentration of about 10% to about 30% w/v *e.g.,* about 15% to about 20% w/v, for blood culture samples. Other suitable materials that can be used to prepare the density cushion include low viscosity, high density oils, such as microscope immersion oil (*e.g.,* Type DF; Cargille Labs, New York), mineral oil (*e.g*., Drakeol® 5, Draketex 50, Peneteck®; Penreco Co., Pennsylvania), silicone oil (polydimethylsiloxane), fluorosilicone oil, silicone gel, metrizoate-Ficoll® (LymphoPrep™), *e.g.,* at a concentration of about 75% to about 100% for blood culture samples, diatrizoate-dextran (PolymorphoPrep™), *e.g.,* at a concentration of about 25% to about 50% for blood culture samples, carboxymethyl cellulose, hydroxypropylmethyl cellulose, polyethylene oxide (high molecular weight), Pluronic® F127, Pluronic® F68, mixtures of Pluronic® compounds, polyacrylic acid, cross-linked polyvinyl alcohol, cross-linked polyvinyl pyrrolidine, PEG methyl ether methacrylate, pectin, agarose, xanthan, gellan, Phytagel®, sorbitol, Ficoll® (*e.g.*, Ficoll® 400 at a concentration of about 10% to about 15% for blood culture samples), glycerol, dextran (*e.g.,* at a concentration of about 10% to about 15% for blood culture samples), glycogen, cesium chloride (*e.g*., at a concentration of about 15% to about 25% for blood culture samples), perfluorocarbon fluids (*e.g*., perfluoro-n-octane), hydrofluorocarbon fluids (*e.g*., Vertrel XF), and the like as are well known in the art. In one example, the density cushion can be selected from one or more of colloidal silica, iodixanol, iohexol, cesium chloride, metrizoate-Ficoll®, diatrizoate-dextran, sucrose, Ficoll® 400, and/or dextran in any combination. The density cushion can also be made up of a combination of materials, *e.g.,* a combination of colloidal silica and oil. Certain combinations of the above compounds may be beneficial for the separation and reading steps of the present invention. For example, combinations of compounds with different UV-quenching properties, such as cesium chloride and Iohexol.

The volume/height of the density cushion should be sufficient to achieve separation of the microorganisms from other sample components. The volume will depend on the size and shape of the separation container. In general, a volume of about 0.1 to about 5 ml can be used, *e.g.,* about 0.2 to about 1 ml, *e.g.,* about 0.2 ml to about 0.5 ml. If the separation is performed on a microscale, the volume of the density cushion can be about 1 µl to about 100 µl, *e.g.,* about 5 µl to about 50 µl. The volume of sample laid or layered on top of the density cushion should be sufficient to provide enough microorganisms to produce a pellet suitable for interrogation. In general, any volume that fits into the container can be used. For example, a volume of about 0.1 ml to about 5 ml can be used, *e.g.,* about 0.2 ml to about 1 ml, *e.g.,* about 0.2 ml to about 0.5 ml. If the separation is performed on a microscale, the volume of sample can be about 1 µl to about 100 µl, *e.g.,* about 5 µl to about 50 µl. The available space in the container for sample will depend on the size and shape of the container. According to the invention, an intermediate layer (liquid or solid) is placed on top of the density cushion before the sample is laid or layered on top in order to prevent any mixing of the density cushion and the sample; wherein the intermediate layer is of polyethylene beads. In a further embodiment, the density cushion can be layered on top of a high density material (*e.g.,* a perfluorocarbon fluid) such that the microorganisms pass through the density cushion during the separation and collect at the interface between the density cushion and the high density material.

The separation container can be centrifuged in a swing out rotor so that the microorganisms form a pellet directly on the bottom of the container. The container is centrifuged at a sufficient acceleration and for a sufficient time for the microorganisms to be separated (e.g., a pellet formed) from other components of the sample. The centrifugation acceleration can be about 1,000 x *g* to about 20,000 x *g*, *e.g.,* about 2,500 x *g* to about 15,000 x *g, e.g.,* about 7,500 x *g* to about 12,500 x *g, etc.* The centrifugation time can be about 30 seconds to about 30 minutes, *e.g.,* about 1 minute to about 15 minutes, *e.g.,* about 1 minute to about 5 minutes. The centrifugation can be carried out at a temperature of about 2°C to about 45°C, *e.g.,* about 15°C to about 40°C, *e.g.,* about 20°C to about 30°C. The separation container comprises a closure, and the closure can be applied to the container to form a hermetic seal prior to centrifugation. The presence of a closure decreases the risks from handling microorganisms that are or may be infectious and/or hazardous, as well as the risk of contaminating the sample. One of the advantages of the methods of the invention is the ability to carry out any one or more of the steps of the methods (*e.g*., lysis, separation, interrogation, and/or identification) with the microorganisms in a sealed container (*e.g*., a hermetically sealed container). Methods, described herein, involve the use of automated systems, avoid the health and safety risks associated with handling of highly virulent microorganisms, such as occurs with recovery of microorganisms from samples for direct testing.

Once the separated, isolated or pelleted microorganism sample has been prepared, a subsequent interrogation step can be carried out to provide measurements useful for characterization and/or identification of the microorganism. Useful interrogation means are known in the art. Additional interrogation means are described in the commonly assigned U.S. patent applications discussed hereinabove.

### EXAMPLES

### EXAMPLE 1. Devices and Methods for the in situ Identification of Purified Microbial Pellet

To explore the potential of the rapid *in situ* separation and identification of microorganisms in a separation device, several devices were designed and molded from UV-transparent plastic, in accordance with this invention. These devices contained several common features, including a closure, sample reservoir and a tapered optical quality lower region to enable spectroscopic interrogation of the sedimented microbial pellet from below and/or the side, and features that facilitated the coupling of the device to a spectrofluorimeter. The devices must also be capable of withstanding relatively high g-forces during the separation step. Several iterations of this tube were designed to improve microbial recovery, fluorescence reproducibility and reduce contamination by stray scattered light. The tube was also designed to be hermetically sealed.

Optical interrogation of the sedimented microbial pellet was achieved by either inserting the separation device into a custom-built adapter placed within the sample compartment of the spectrofluorimeter or by coupling the separation device directly to a bifurcated six-around-one 300-400 micron fiber optic cable (Ocean Optics, Dunedin, FL) attached to the spectrofluorimeter (Fluorolog® 3 from HORIBA Jobin Yvon Inc., New Jersey). A three-mirror fiber optic adapter was built to enable the use of both the systems detectors (PMT and CCD). Full Excitation-Emission Matrix (EEM) spectra were collected on each microbial pellet (scan range: Excitation 260-800 nm; Emission 260-1100 nm; increments of 5 nm).

Gage reproducibility and reliability studies were performed on the disposable device-fiber optic cable configuration using purified tryptophan and riboflavin solutions. Target CV's of < 2.5% were obtained for both fluorophores, confirming the quality of the disposable and the research platform.

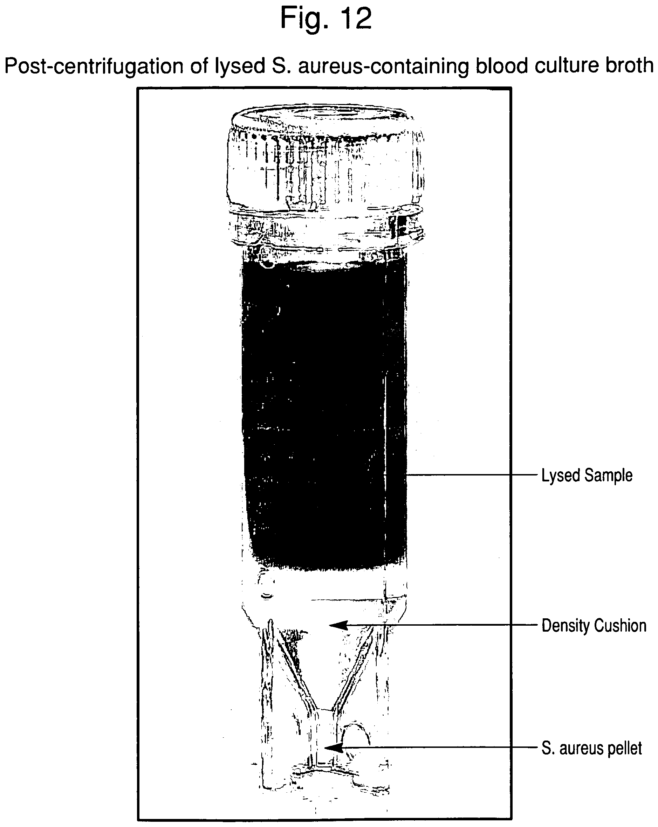

These devices proved useful for the separation and interrogation of microorganisms from a culture medium. Figure 12 shows an example device after separation by centrifugation of a lysed blood culture sample containing *S*. *aureus* using a density cushion. Clearly visible in the photograph are the lysed sample, density cushion and a microorganism pellet.

## Claims

1. A container for isolating and identifying a microorganism, said container comprising:
(a) a lower portion comprising a capillary tube having an internal diameter from 0.001 to 0.04 inches (0.0254 mm to 1.016 mm);
(b) an upper internal chamber or reservoir having an internal diameter wider than the internal diameter of said capillary tube;
(c) a middle tapered portion connecting said upper portion to said lower portion;
(d) a density cushion in the container, wherein the density cushion has a homogeneous density in the range of 1.025 to 1.120 g/ml, wherein an intermediate layer of polyethylene beads is placed on top of the density cushion, and wherein the container further contains a selective lysis solution;
(e) an optical window on the bottom or one or more sides of the container and having a thickness of less than 0.10 inches (2.54 mm), said optical window being transparent to at least a portion of the near infrared, visible, and/or ultraviolet light spectrum and wherein said optical window comprises quartz, fused silica, sapphire, acrylic, methacrylate, a cyclic olefin copolymer, a cyclo olefin polymer; and
(f) a closure or cap.

2. The container of claim 1, wherein said lower portion is less than half of the total height of the container.

3. The container of claim 1 or 2 , wherein said container is threaded to accept the closure device such that the container can be sealed.

## Patentansprüche

1. Ein Behälter zur Isolierung und Identifikation eines Mikroorganismus, wobei der Behälter Folgendes beinhaltet:
(a) einen unteren Abschnitt, der ein Kapillarröhrchen mit einem Innendurchmesser von 0,001 bis 0,04 Zoll (0,0254 mm bis 1,016 mm) beinhaltet;
(b) eine obere innere Kammer oder ein oberes inneres Reservoir mit einem Innendurchmesser, der weiter ist als der Innendurchmesser des Kapillarröhrchens;
(c) einen mittleren, sich verjüngenden Abschnitt, der den oberen Abschnitt mit dem unteren Abschnitt verbindet;
(d) ein Dichtepolster in dem Behälter, wobei das Dichtepolster eine homogene Dichte im Bereich von 1,025 bis 1,120 g/ml aufweist, wobei eine Zwischenschicht von Polyethylenkügelchen auf der Oberseite des Dichtepolsters platziert ist und wobei der Behälter ferner eine selektive Lyselösung enthält;
(e) ein optisches Fenster auf der Unterseite oder einer oder mehreren Seiten des Behälters und mit einer Dicke von weniger als 0,10 Zoll (2,54 mm), wobei das optische Fenster für mindestens einen Teil des nahinfraroten, sichtbaren und/oder ultravioletten Lichtspektrums transparent ist und wobei das optische Fenster Quarz, Kieselglas, Saphir, Acryl, Methacrylat, ein Cycloolefin-Copolymer, ein Cycloolefin-Polymer beinhaltet; und
(f) einen Verschluss oder eine Kappe.

2. Behälter gemäß Anspruch 1, wobei der untere Abschnitt weniger als die Hälfte der Gesamthöhe des Behälters ausmacht.

3. Behälter gemäß Anspruch 1 oder 2, wobei der Behälter ein Gewinde zum Aufnehmen der Verschlussvorrichtung aufweist, sodass der Behälter versiegelt werden kann.

## Revendications

1. Un récipient pour isoler et identifier un micro-organisme, ledit récipient comprenant :
(a) une partie inférieure comprenant un tube capillaire ayant un diamètre intérieur allant de 0,001 à 0,04 pouce (0,0254 mm à 1,016 mm) ;
(b) une chambre ou un réservoir intérieur(e) supérieur(e) ayant un diamètre intérieur plus large que le diamètre intérieur dudit tube capillaire ;
(c) une partie conique centrale reliant ladite partie supérieure à ladite partie inférieure ;
(d) un coussin de densité dans le récipient, dans lequel le coussin de densité a une masse volumique homogène dans la plage allant de 1,025 à 1,120 g/ml, dans lequel une couche intermédiaire de billes de polyéthylène est placée au-dessus du coussin de densité, et dans lequel le récipient contient en outre une solution de lyse sélective ;
(e) une fenêtre optique sur le fond ou sur un ou plusieurs côtés du récipient et ayant une épaisseur de moins de 0,10 pouce (2,54 mm), ladite fenêtre optique étant transparente à au moins une partie du spectre de lumière infrarouge proche, visible et/ou ultraviolette et dans lequel ladite fenêtre optique comprend du quartz, de la silice fondue, du saphir, de l'acrylique, du méthacrylate, un copolymère d'oléfine cyclique, un polymère cyclo-oléfine ; et
(f) une fermeture ou un bouchon.

2. Le récipient de la revendication 1, dans lequel ladite partie inférieure fait moins de la moitié de la hauteur totale du récipient.

3. Le récipient de la revendication 1 ou de la revendication 2, dans lequel ledit récipient est fileté afin de recevoir le dispositif de fermeture de telle sorte que le récipient puisse être fermé hermétiquement.
